Europäisches Patentamt

European Patent Office

Office européen des brevets

(19)

(11) Publication number: **0 041 784**
**A1**

(12) **EUROPEAN PATENT APPLICATION**

(21) Application number: **81302189.6**

(22) Date of filing: **18.05.81**

(51) Int. Cl.$^3$: **C 07 C 63/26**
C 07 C 51/255, C 07 C 51/265

(30) Priority: **10.06.80 GB 8018914**

(43) Date of publication of application:
**16.12.81 Bulletin 81/50**

(84) Designated Contracting States:
**BE DE FR GB IT NL**

(71) Applicant: IMPERIAL CHEMICAL INDUSTRIES LIMITED
*Imperial Chemical House Millbank*
**London SW1P 3JF(GB)**

(72) Inventor: **Vynne Norval, Stephen**
**3 Merlin Close**
**Guisborough Cleveland(GB)**

(72) Inventor: **Heath Cowles, Roger John**
**115 Farndale Drive Pine Hills**
**Guisborough Cleveland(GB)**

(74) Representative: **Taylor, Michael et al,**
**Imperial Chemical Industries Limited Legal Department:**
**Patents Thames House North Millbank**
**London SW1P 4QG(GB)**

(54) **Oxidation of substituted aromatic compounds to aromatic carboxylic acids.**

(57) Oxidation of a substituted aromatic compound, e.g. p-xylene, to an aromatic carboxylic acid, e.g. terephthalic acid, in an essentially aqueous reaction medium at 200-300°C, using as catalyst manganese alone or manganese and a lesser amount of another heavy metal (cobalt and/or nickel).

EP 0 041 784 A1

Croydon Printing Company Ltd.

## Oxidation of Substituted Aromatic Compounds to Aromatic Carboxylic Acids

THE PRESENT INVENTION relates to the oxidation of substituted aromatic compounds to aromatic carboxylic acids.

Aromatic carboxylic acids are important chemical products, the dibasic acids in particular finding a major outlet in the resin and plastic industry. Terephthalic acid and isophthalic acid are important examples of this latter class of compounds and they are commonly produced by the oxidation of the corresponding xylene (p-xylene and m-xylene respectively). The oxidation is carried out using molecular oxygen in the presence of a catalyst, the most widely used being a heavy metal such as cobalt in conjunction with bromine or a bromine-containing compound. The solvent for the catalyst and reactants is usually a lower alkanoic acid, acetic acid being almost universally used. An alkanoic acid such as acetic acid although providing an effective solvent has the disadvantage of susceptibility to oxidation which means that solvent is lost as oxides of carbon and must be replaced. The replacement is a significant item in the overall cost of the process.

It would be a substantial step forward in the technology of aromatic carboxylic acid production if water could be used as all or as a substantial part of the reaction medium because it is cheap and not susceptible to oxidation. Unfortunately, under the reaction conditions and with the catalyst usually used in the acetic acid-solvent process the use of water as solvent gives a process with very poor yield of an inferior product. The effect of the present invention is so to select the conditions under which the process is operated, particularly with respect to temperature and choice of catalyst, that water may be used as solvent and an acceptable product obtained.

Accordingly, the invention is a process for the production of an aromatic carboxylic acid which comprises oxidising by means of molecular oxygen a substituted aromatic compound in the presence of a catalyst comprising a heavy metal compound and bromine or a bromine-containing compound, characterised in that the oxidation is carried out in an essentially aqueous reaction medium at a temperature in the range 200 to 300°C and using a catalyst comprising manganese alone or manganese and a lesser amount of one or more other heavy metals.

Preferably the temperature is 210°C to 260°C. The atomic ratio of manganese to other heavy metals is preferably 3:1 to 20:1 more preferably 4:1 to 10:1. The amount of manganese present during the oxidation is subject to wide variation but may lie in the range 20 to 20,000 ppm based on solvent preferably 1000 to 10,000 ppm. The amounts of any heavy metals other than manganese which may be present e.g. vanadium, chromium, iron, molybdenum, a lanthanide e.g. cerium, zirconium, hafnium and, particularly, cobalt and/or nickel may lie in the range 10 to 6,000 ppm based on solvent, particularly 100 to 2,000 ppm. The heavy metals may be used, for example, in the form of their inorganic or organic acid salts especially the bromides or lower ($C_1$ to $C_4$) alkanoates e.g. acetates.

The catalyst also comprises bromine or a bromine-containing compound which acts as an oxidation promoter. The bromine may be provided as bromine itself, as hydrogen bromide, as an organic bromine compound e.g. tetrabromoethane or as an inorganic bromide. Suitable bromides, include, for example, bromides of manganese and/or other heavy metal(s). The amount of bromine present in the process may vary widely but generally lies in the range 500 to 50,000 ppm based on the weight of solvent. Preferably the bromine is provided as hydrogen bromide.

The solvent used in the process is essentially water. When p-xylene is the substituted aromatic compound terephthalic acid may be provided as solubilising agent before the start of the oxidation reaction but as the reaction proceeds the terephthalic acid which is produced fulfills this function.

The pressure under which the process is operated is at least such that a liquid phase is maintained in the reactor and is, for example, up to 200 bar preferably 15 to 150 bar.

The molecular oxygen used in the process may be used alone or in admixture with other gases e.g. as air or as a mixture of oxygen and nitrogen with a higher or lower oxygen content than that of air.

The aromatic compound is preferably substituted by an alkyl, hyroxyalkyl, or a formyl group. Particularly suitable alkyl groups are lower ($C_1$ to $C_8$) alkyl groups e.g. methyl, ethyl and isopropyl groups. Particularly suitable hydroxyalkyl groups are hydroxymethyl groups.   One, two or more such groups may be present in the aromatic nucleus and the groups may be the same of different.   The aromatic nucleus may, for example, be a benzene or naphthalene . nucleus.   Particularly suitable aromatic compounds to be oxidised are toluene, ethylbenzene, isopropylbenzene, o-, m- and p-xylene, cumene, pseudocumene, the isomeric diisopropylbenzenes, durene, mesitylene, hydroxymethylbenzene, hydroxyethylbenzene, bis-hydroxy-methylbenzenes, benzaldehyde and 2,6 -dimethylnaphthalene.   Suitable aromatic compounds also include those which are already partially oxidised to carboxylic acids and their corresponding esters, for example, the isomeric tolualdehydes,p-toluic acid, methyl p-toluate and p-carboxybenzaldehyde.   The process of our invention is particularly suitable for the oxidation of p-xylene, p-tolualdehyde or p-toluic acid to terephthalic acid.

The separation and purification of the aromatic dibasic acid product of the process may be carried out by known means.   Thus the product may be cooled, separated by filtration or by centrifuging and washed with fresh solvent.   It may also be subject to a secondary or post-oxidation to improve its quality and/or it may be catalytically hydrogenated in the presence of a catalyst e.g. platinum to achieve the same object.

The invention will now be further described with reference to the following examples in which the apparatus used comprised a titanium autoclave of total capacity 4.5 litres, equipped with a heater, reflux condenser, agitator, gas inlet and outlet, and feed inlet and outlet.   The purity of the terephthalic acid product of the process described in the Examples was assessed by measuring its 4-carboxybenzaldehyde content, the latter compound being an intermediate oxidation product.

EXAMPLES 1-9

54 g p-toluic acid was charged to the autoclave along with 1800 ml water, 9.2 ml of 47% w/w aqueous HBr and various amounts of cobalt dibromide hexahydrate, manganese dibromide tetrahydrate and nickel dibromide trihydrate to give the metal concentrations indicated in the table.   The reaction mixture was then heated under a nitrogen atmosphere to 215°C and a total pressure of 34.5 bar.   A mixture of 30% oxygen in nitrogen was then passed through the autoclave at a total flow rate of about 700 1 per hour (measured at STP). After 30 minutes oxidation, a small sample of the reaction mixture was removed from the autoclave. The oxidation was continued for a further 30 minutes and another sample of reaction mixture removed from the autoclave. The samples of reaction mixture were cooled to ambient temperature and the product filtered, washed with water and dried. The dried products were analysed and found to contain the 4-carboxybenzaldehyde (4CBA) levels given in the table.

| Example No. | Catalyst Metal Levels in Mother Liquor | | | 4CBA Content of TA Product | |
|---|---|---|---|---|---|
| | Mn (ppm) | Co (ppm) | Ni (ppm) | After 30 min oxidation (wt. %) | After 60 min oxidation (wt. %) |
| 1 | 1063 | 188 | 0 | 0.62 | 0.25 |
| 2 | 1000 | 250 | 0 | 1.5 | 0.62 |
| 3 | 625 | 625 | 0 | 3.7 | 1.5 |
| 4 | 1125 | 0 | 125 | 0.78 | 0.41 |
| 5 | 938 | 0. | 312 | 1.0 | 0.85 |
| 6 | 625 | 0 | 625 | 3.0 | 1.5 |
| 7 | 1250 | 0 | 0 | 3.6 | |
| 8 | 0 | 1250 | 0 | 2.6 | |
| 9 | 0 | 0 | 1250 | 6.4 | |

EXAMPLE 10

The autoclave was charged with 1800 ml water, 16.9 g of manganese dibromide tetrahydrate and 12.4 ml of 47% w/w aqueous HBr. The reactor contents were heated to 230°C at a total pressure of 38 bar under a nitrogen gas flow. A flow of approximately 20% oxygen in nitrogen was then substituted for the nitrogen flow and 180 ml p-tolualdehyde (pTAL) introduced continuously to the reactor over a period of one hour. A sample of the reaction mixture was then removed from the reactor, cooled to ambient temperature and the solid product filtered, washed with water and dried. The terephthalic acid product was found to contain 1.45% by weight of 4CBA.

EXAMPLE 11

The experiment of Example 10 was repeated, except that the catalyst added comprised 1.88 g of cobalt dibromide hexahydrate 10.0 g of manganese dibromide tetrahydrate and 9.2 ml of 47% w/w aqueous HBr, and that 250 ml pTAL was introduced to the reactor continuously over one hour. The terephthalic acid product was found to contain 0.68% by weight of 4CBA.

EXAMPLE 12

The autoclave was charged with 1800 ml water, 11.7 g of manganese dibromide tetrahydrate, 0.12 g of potassium iodide and 13.2 ml of 47% w/w aqueous HBr. The reactor contents were heated to 246°C at a total pressure of 56 bar under a nitrogen gas flow. A flow of approximately 20% oxygen in nitrogen was then substituted for the nitrogen flow and 240 ml pTAL introduced continuously to the reactor over a period of one hour. The pTAL feed to the reactor having ceased, the oxygen and nitrogen flow was continued for a further 30 minutes.while the temperature was maintained at 246°C. The reactor contents were then cooled under a nitrogen flow to about 150°C and solid terephthalic acid product filtered off, washed in water and dried. The resulting terephthalic acid product contained 0.11% by weight 4CBA and 0.16% by weight pTA.

EXAMPLE 13

The experiment of example 12 was repeated except that the catalyst added comprised 10.0g manganese dibromide tetrahydrate 1.88 g cobalt dibromide hexahydrate,0.12 g potassium iodide and 8.9 ml of 47% w/w aqueous HBr. 309 g of terephthalic acid product was recovered, which contained 0.02% by weight 4CBA and 0.03% by weight pTA.

EXAMPLE 14

The autoclave was charged with 1800 ml water, 90 g purified terephthalic acid, 10.0 g manganese dibromide tetrahydrate, 1.88 g cobalt dibromide hexahydrate, 0.12 g potassium iodide and 8.9 ml 47% w/w aqueous HBr. The reactor contents were heated to 248°C at a total pressure of 56 bar under a nitrogen gas flow. A flow of approximately 20% oxygen in nitrogen was then substituted for the nitrogen flow and 62 ml of p-xylene was introduced continuously to the reactor over a period of one hour. The p-xylene flow having ceased, the oxygen and nitrogen flow was continued for a further 30 minutes while the temperature was maintained at 248°C. The reactor contents were then cooled to about 150°C under nitrogen flow and the solid terephthalic acid product filtered off, washed in water and dried.   The 164 g of terephthalic acid recovered contained 0.05% by weight 4CBA and 0.10% by weight pTA.

EXAMPLE 15

The autoclave was charged with 1800 ml water, 11.7 g manganese dibromide tetrahydrate and 8.9 ml of 47% by weight of aqueous HBr. The reactor contents were heated to 246°C at a total pressure of 56 bar under a nitrogen gas flow.   A flow of approximately 20% oxygen in nitrogen was then substituted for the nitrogen flow and 85 ml of p-tolualdehyde introduced .continuously to the reactor over a period of 20 minutes.   Immediately following this p-tolualdehyde flow, 60 ml p-xylene was introduced continuously to the reactor over a period of 1 hour.   The p-xylene flow having ceased, the oxygen and nitrogen flow was continued for a further 30 minutes while the temperature was maintained at 246°C.   The reactor contents were then cooled under a nitrogen flow to about 150°C and the solid terephthalic acid product filtered off, washed in water and dried. The resulting terephthalic acid product contained 0.15% by weight 4CBA and 0.15% by weight pTA.

Claims

1.      A process for the production of an aromatic carboxylic acid which comprises oxidising by means of molecular oxygen a substituted aromatic compound in the presence of a catalyst comprising a heavy metal compound and bromine or a bromine-containing compound, characterised in that the oxidation is carried out in an essentially aqueous reaction medium at a temperature in the range 200 to 300°C. and using a catalyst comprising manganese alone or manganese and a lesser amount of one or more heavy metals.

2.      A process according to Claim 1 characterised in that the temperature is 210°C to 260°C.

3.      A process according to Claim 1 or Claim 2 characterised in that the atomic ratio of manganese to other heavy metals is 3:1 to 20:1.

4.      A process according to Claim 3 characterised in that the ratio is 4:1 to 10:1.

5.      A process according to any one of the preceding claims characterised in that the amount of manganese present is in the range 20 to 20,000 ppm based on solvent.

6.      A process according to any one of the preceding claims characterised in that cobalt and/or nickel are also present.

7.      A process according to any one of the preceding claims characterised in that the bromine-containing compound is hydrogen bromide or a bromide of manganese and/or other heavy metal(s).

8.      A process according to any one of the preceding claims characterised in that the substituted aromatic compound comprises p-xylene, p-tolualdehyde, p-toluic acid, methyl p-toluate or p-carboxybenzaldehyde.

9.      A process according to Claim 8 characterised in that the substituted aromatic compound is p-xylene and terephthalic acid is provided as solubilising agent before the start of the oxidation reaction.

MT/CB

0041784

Application number

EP 81 30 2189

## DOCUMENTS CONSIDERED TO BE RELEVANT

| Category | Citation of document with indication, where appropriate, of relevant passages | Relevant to claim |
|---|---|---|
| X | GB - A - 2 025 956 (MITSUBISHI GAS)<br>* Claims 1,2,9,10,12 *<br><br>-- | 1,2,7,8 |
| X | GB - A - 2 009 735 (MITSUBISHI GAS)<br>* Claims 1,2,6,7,10 *<br><br>-- | 1,2,7,8 |
| X | US - A - 3 012 038 (O'NEILL et al.)<br>* Claims 1-3; column 2, lines 18,19 *<br><br>-- | 1-4,6-9 |
| | GB - A - 2 019 395 (LABOFINA)<br>* Claim 1; page 1, lines 121-125 *<br><br>---- | 1,2,6,8,9 |

### CLASSIFICATION OF THE APPLICATION (Int. Cl.³)

C 07 C 63/26
51/255
51/265

### TECHNICAL FIELDS SEARCHED (Int. Cl.³)

C 07 C 63/26
51/255
51/265

### CATEGORY OF CITED DOCUMENTS

X: particularly relevant

A: technological background

O: non-written disclosure

P: intermediate document

T: theory or principle underlying the invention

E: conflicting application

D: document cited in the application

L: citation for other reasons

&: member of the same patent family, corresponding document

The present search report has been drawn up for all claims

| Place of search | Date of completion of the search | Examiner |
|---|---|---|
| The Hague | 11-09-1981 | KLAG |

EPO Form 1503.1  06.78